# EUROPÄISCHE PATENTSCHRIFT

(11) **EP 0 338 518 B1**
(45) Veröffentlichungstag und Bekanntmachung des Hinweises auf die Patenterteilung: **23.06.1993**
(21) Anmeldenummer: 89106949.4
(22) Anmeldetag: 19.04.1989
(51) Int. Cl.: A61M 16/14, A61M 16/18

(54) **Narkoseeinrichtung**
Anaesthetic equipment
Dispositif d'anesthésie

(30) Priorität: 22.04.1988 DE 3813520
(43) Veröffentlichungstag der Anmeldung: 25.10.1989
(73) Patentinhaber: Drägerwerk Aktiengesellschaft, 23542 Lübeck (DE)
(72) Erfinder: Altner, Ulrich, Dr. Dipl.-Ing., D-2360 Bad Segeberg (DE); Brandt, Claus-Dieter, Dr. Dipl.-Ing., D-2407 Bad Schwartau (DE); Buschke, Wilfried, Dipl.-Ing., D-2400 Lübeck (DE); Falb, Wolfgang, Dipl.-Ing., D-2061 Klein Wesenberg (DE); Kullik, Götz, Dipl.-Ing., D-2400 Lübeck (DE); Schwanbom, Erik, Dr. Dipl.-Chem., D-2400 Lübeck (DE); Wallroth, Carl Friedrich, Dr. Dipl.-Ing., D-2400 Lübeck (DE)

(56) Entgegenhaltungen:
- DE-A- 2 243 733
- DE-C- 3 222 047
- GB-A- 2 177 007
- US-A- 4 463 754

## Beschreibung

Die Erfindung betrifft eine Narkoseeinrichtung gemäß dem ersten Teil von Anspruch 1.

Eine derartige Narkoseeinrichtung ist aus der DE-OS 35 23 947 bekannt geworden.

Bei der bekannten Narkoseeinrichtung ist ein Vorratsbehälter für ein Narkosemittel an eine zugehörige Einstellvorrichtung angeschlossen, wobei mittels geeigneter Kodierstifte der Vorratsbehälter an die Einstellvorrichtung derart eingreift, daß eine Dosierung des Narkosemittels auf die jeweiligen, die Dosierung beeinflussenden physikalischen Eigenschaften des Narkosemittels abgestimmt ist. Der Verdunster und die Einstellvorrichtung werden an ein dazugehöriges Narkosegerät angeschlossen, so daß das dosierte Narkosemittel an das Narkosegerät abgegeben werden kann. Für eine bestimmungsgemäße Verwendung des Narkosegerätes muß das für die jeweilige Narkose erforderliche Narkosemittel auch tatsächlich in dem dazugehörigen Verdunster und der Einstellvorrichtung angeschlossen sein. Im Laufe einer Narkosedurchführung ist es manchmal darüber hinaus erforderlich, die Narkosemittel zu wechseln. Dazu muß entweder der angeschlossene Verdunster durch einen anderen ersetzt, oder bei mehreren an das Narkosegerät angeschlossenen Verdunstern von einem zum anderen Verdunster gewechselt werden. Dabei ist es unerläßlich, daß der Benutzer des Narkosegerätes sich Sicherheit verschafft, ob er den gewünschten Verdunster auch tatsächlich angeschlossen und eingeschaltet hat. Dies erfordert von dem Narkosearzt erhöhte Aufmerksamkeit, die durch eine Belastung während einer beispielsweise länger andauernden Narkose nicht immer gleichmäßig gegeben ist. Es besteht zwar die Möglichkeit, das verabreichte Narkosemittel durch ein Meßgerät zu kontrollieren, doch besitzen nur sehr aufwendige Meßgeräte die Fähigkeit, aus der Messung selbst zugleich die Art des verwendeten Narkosemittels festzustellen und daraus für die Anzeige die passende Eichkurve anzuwenden. Einfachere Meßgeräte bedürfen dazu einer manuellen Umschaltung auf die Art des vorliegenden Narkosemittels, was bei irrtümlichen Schaltfehlern oder einer bei Wechsel des Narkosemittels unterlassenen Anpassung zu Fehlmessung und Verabreichung falscher Dosierungen führen kann.

Der vorliegenden Erfindung liegt somit die Aufgabe zugrunde, eine Narkoseeinrichtung der genannten Art so zu verbessern, daß mit einfachen Mitteln eine automatische Erkennung des jeweiligen benutzten Narkosemittels und der dazugehörigen Einstellvorrichtung schon bei deren Anschluß an das Narkosegerät sichergestellt ist und daß diese Erkennung einem entsprechenden Meß- und Überwachungsgerät für das Narkosemittel weitergeleitet wird, um Fehlmessungen und Fehleinstellungen zu vermeiden oder auch die Voraussetzung für eine selbsttätige Einhaltung einer gewünschten Konzentration zu schaffen.

Die Lösung der Aufgabe erfolgt durch die im Anspruch 1 definierte Einrichtung.

Der Vorteil der Erfindung liegt im wesentlichen darin, daß jeder der anzuwendenden Narkosemittelverdunster mit der dazugehörigen Einstellvorrichtung die Information über Art des Narkosemittels, des möglichen Einstellbereiches für die Dosierung und weitere, narkosemittelspezifische Daten an die Meß- und Überwachungseinheit weitergeben kann. Der Versuch, einen Narkosemittelverdunster mit ungeeignetem bzw. nicht gewünschtem Inhalt anzuschließen, kann entweder verhindert oder auch als Fehlbedienung kenntlich gemacht werden.

Vorteilhaft ist ferner eine Kompatibilität mit bauähnlichen Verdunstern, die jedoch die erfindungsgemäße Kennung noch nicht besitzen. Die fehlende Kennung führt an der Meß- und
Überwachungseinheit zu einer Alarmgabe, die für den Fall der Benutzung deren Ausnahmecharakter bewußt macht.

Durch die einfache Art der an der Einstellvorrichtung angeordneten Kennungselemente besteht außerdem die Möglichkeit, mit geringem Aufwand eine Nachrüstung vorhandener, diese Elemente noch nicht besitzender Geräte durchzuführen.

Die Meß- und Überwachungseinheit wird durch die erkannte Kodierung entweder selbsttätig auf die dem betreffenden Narkosemittel zugehörige Eichkurve umgeschaltet oder es wird bei Handbetätigung die vorgenommene Bereichswahl kontrolliert. Einer falschen Bereichseinstellung durch Irrtum oder vergessene Umschaltung und damit Anzeige fehlerhafter Konzentrationsangaben ist dadurch vorgebeugt.

Mit geringem Aufwand kann die Anordnung zu einem geschlossenen Regelkreis vervollständigt werden, indem der Meß- und Überwachungseinheit eine Sollwerteingabe und ein Stellglied zugeordnet werden, das z.B. mittels einer Verzahnung lösbar in die Einstellvorrichtung eingreift. Auch hier werden durch die Kodierung die Werte dem jeweils angeschlossenen bzw. eingeschalteten Narkosemittel angepaßt und Fehlbedienung vermieden.

Von den verschiedenen möglichen Ausführungsformen der Kennung hat sich als besonders zweckmäßig erwiesen, ein Kupplungsstück zwischen dem Verdunster und der Einstellvorrichtung einerseits und dem Narkosegerät andererseits vorzusehen, in das mehrere Ausnehmungen unterschiedlicher Gestaltung eingelassen sind. Dieses Kupplungsstück kann z. B. am Narkosegerät angebracht sein, in welches der Verdunster und die Einstellvorrichtung eingehängt werden. Dabei greifen ein oder auch mehrere Paßteile in die ihnen zugeordneten Ausnehmungen ein, wobei die äußere Form der Paßteile an die äußere Gestaltung der Ausnehmungen angeglichen ist. Die Form und Ausgestaltung zwischen Ausnehmungen und Paßteilen kann ein unerlaubtes Anschließen eines Verdunsters an das Narkosegerät verhindern. Beispielsweise soll ein Verdunster mit vier Paßteilen an ein Kupplungsstück mit nur drei Ausnehmungen angeschlossen werden. Da das vierte Paßteil keine Entsprechung im Kupplungsstück findet, wird eine paßgenaue Montage verhindert. Die Abtastvorrichtung erkennt diesen Fehlversuch durch die Signalgeber und gibt eine entsprechende Meldung an die Meß- und Überwachungseinheit ab.

Eine einfache Ausführungsform wird dadurch verwirklicht, daß die Ausnehmungen als Bohrungen unterschiedlicher Tiefe ausgebildet sind, in welcher entsprechende Stifte eingreifen, durch die je nach Eingriffstiefe ein mit ihnen gekoppelter elektrischer Schalter oder eine Lichtschranke als Signalgeber betätigbar ist. Eine derartige Stiftkodierung erlaubt es, je nach Eindringtiefe der Stifte in die Bohrungen die elektrischen Schalter entweder zu betätigen oder in ihrer Ruhestellung zu belassen, wobei deren entsprechende Signale als Ein- oder Auszustände gewertet werden. Auf diese Weise wird eine einfache digitale Stiftkodierung ermöglicht. Die Variation der Kodiermöglichkeiten ist sowohl mit der Anzahl der Stifte als auch mit ihrer unterschiedlichen Eindringtiefe kombinierbar.

Im Gegensatz zu den vorgenannten mechanischen Kennungen ist eine optische Kennung mit Hilfe einer Anordnung von Reflexlichtschranken vorteilhaft, wobei die Kodierung in einem spezifischen Muster von Reflexmarken übersetzt werden kann, die wahlweise entweder am Narkosegerät oder an der Einstellvorrichtung vorgesehen werden können. Diese werden über eine in ihrem Muster entsprechende Anordnung von Leuchtkörpern angestrahlt, und die Kodierung wird je nach Anzahl der reflektierten Lichtstrahlen an einen Empfänger weitergeleitet. Dort werden sie dekodiert und als entsprechende Information an die Meß- und Überwachungseinheit weitergegeben. Eine derartige Lichtreflexkodierung besitzt den Vorteil, daß sie berührungslos arbeitet, mit einfachen Mitteln verwirklicht werden kann und der mechanische Aufwand dazu möglichst gering gehalten wird.

Als weitere Alternative bietet sich eine magnetische Kodierung an der Berührebene zwischen Aufnahmeblock und Halterung an. Dazu ist zweckmäßigerweise als Abtastvorrichtung ein Magnetstreifenleser vorgesehen, über den beim Ankuppeln der Narkoseeinrichtung an das Narkosegerät ein Magnetstreifen in Position gebracht ist. Um die auf dem Magnetstreifen gesicherten Daten einlesen zu können, wird dieser an einer dafür vorgesehenen Zunge gleitend über den Lesekopf des Magnetstreifenlesers gezogen. Die eingegebenen Daten werden an die Überwachungseinheit weitergegeben.

Will man zusätzlich zu der Gerätekodierung auch noch die Einstellung selbst an der Einstellvorrichtung kodieren und somit überwachbar machen, kann vorgesehen sein, die Kennung als einen digital kodierten Streifen auszubilden, der am äußeren Rand eines tellerförmig ausgebildeten Handrades an der Einstellvorrichtung aufgebracht ist. Seine Kodiermarken werden von einer optischen Abtasteinheit in Reflexionslicht abgelesen und einer Auswerteeinheit zugeführt, die diese Information wiederum an die Meß- und Überwachungseinheit abgibt. Die Kodierung kann Informationen über die Art des Narkosemittels, die Nullstellung und die Betriebsstellungen des Verdunsters enthalten. Alternativ kann die Einstellung mit einem Drehwinkelgeber an der Einstellvorrichtung erfasst werden. Die Einstellung des Handrades wirkt dabei als Eingabe eines Sollwertes, der nicht nur den Verdunster selbst beeinflußt, sondern über die Abtastung an die Meß- und Überwachungseinheit übertragen wird. Dort kann er je nach Ausführung dazu dienen, eine manuell zu vollziehende Umschaltung des Meßgerätes auf das jeweilige Narkosemittel zu kontrollieren oder die Umschaltung selbsttätig auszuführen. Die Abtastung der Nullstellung erlaubt eine Kontrolle, daß im Betrieb des Narkosegerätes das Einschalten des Verdunsters nicht ungewollt vergessen wird und daß außer Betrieb der Verdunster geschlossen ist. Sind mehrere Verdunster an ein Narkosegerät angesetzt, ist damit kontrollierbar, daß jeweils nur einer davon eingeschaltet ist, und dessen Individualität und Art des verwendeten Narkosemittels zu erkennen. Während des Betriebes wird die am Handrad vorgegebene Konzentration mit den Ist-Werten des Meßgerätes verglichen und gewarnt, sobald zwischen beiden eine unzulässige Abweichung auftritt. Die Toleranz kann dabei nach Erfahrungswerten auf die Größe der eingestellten Konzentration abgestimmt und in die Meß- und Überwachungseinheit einprogrammiert sein.

Für den Fall, daß mehrere Einstellvorrichtungen mit ihrer zugehörigen Kennung an das Narkosegerät angeschlossen sind, werden die nicht eingesetzten Verdunster durch eine Sperrung verriegelt und der benutzte Verdunster freigegeben. Der freigegebene Verdunster ist dann über eine mit der Stellung der Sperrung gekoppelte Reflexlichtschranke als solcher der Meß- und Überwachungseinheit kenntlich gemacht.

Zur weiteren Erleichterung der Auswertung für die Kodierung ist vorgesehen, daß an die Meß- und Überwachungseinheit ein Kodierungsspeicher angeschlossen ist. In diesem sind sämtliche erlaubten Kodierungsmuster gespeichert. Bei Anschluß eines benutzten bzw. freigegebenen Verdunsters werden dessen Kodierungssignale über einen Vergleicher mit dem gespeicherten erlaubten Kodierungsmuster verglichen und überprüft, ob das gelieferte Kodierungsmuster in dem Speichervorrat vorhanden ist. Dadurch wird die Kontrolle und Überwachung bei Verwendung mehrerer Narkosemittel erleichtert.

In einem weiteren Ausführungsbeispiel ist ein Teil des Kodierungsspeichers der Überwachungseinrichtung in der Einstellvorrichtung des Verdunsters angeordnet. Er ist vorteilhaft als Permanentspeicher, z.B. EEPROM, ausgeführt und enthält alle relevanten verdunsterbezogenen Daten wie beispielsweise Kalibrierung, Narkosemittelkennung sowie Fertigungs- und Serviceinformationen. Wird die Einstellung der Einstellvorrichtung z.B. in bekannter Weise mit einem Drehwinkelgeber erfasst, läßt sich dieses Signal zusammen mit den verdunsterbezogenen Daten in einem, ebenfalls in der Einstellvorrichtung angeordneten Mikroprozessor zusammenführen. Der Mikroprozessor steht beispielsweise über eine Signalleitung in Wirkverbindung mit der Überwachungseinheit. Der mit diesem Ausführungsbeispiel erzielte Vorteil liegt hauptsächlich darin, daß nun auch die verdunsterbezogenen, individuellen Kalibrierwerte in den Plausibilitätsvergleich der mit der Einstellvorrichtung vorgegebenen Konzentration und den Istwerten des Meßgerätes einbezogen werden.

Ein Ausführungsbeispiel der Erfindung wird anhand einer schematischen Zeichnung dargestellt und im folgenden näher erläutert.

Es zeigen
- Figur 1: die Ansicht eines an ein Narkosegerät angekoppelten Verdunsters,
- Figur 2: die Teilansicht einer Reflexlichtschrankenkodierung,
- Figur 3: die Draufsicht eines Aufnehmers für eine Stiftkodierung,
- Figur 4: die Ansicht einer Kennung in Form einer Stiftkodierung,
- Figur 5: die Draufsicht auf zwei an ein Narkosegerät angeschlossene Verdunster.

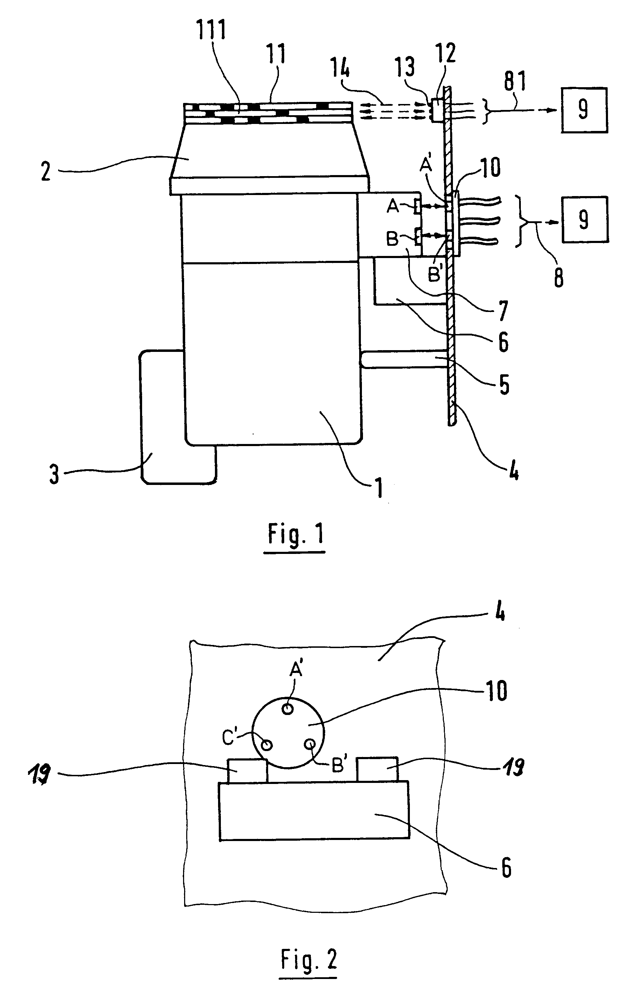

In Figur 1 ist ein Narkosemittelverdunster (1) mit einer dazugehörigen Einstellvorrichtung (2) und einer Narkosemitteleinfüllung (3) dargestellt, dessen Aufnehmer A, B, C mit den ihnen gegenüberliegenden, in Figur 2 vollständig erkennbaren Reflexlichtschranken A', B', C' als Kennung in Wirkverbindung gebracht sind. Zur mechanischen Kopplung und Abstützung an das nur in einem Wandteilbereich dargestellte Narkosegerät (4) ist eine Stütze (5) mit einem Aufnahmeblock (6) am Narkosegerät (4), und eine Halterung (7) am Verdunster (1) vorgesehen. Die von den Reflexlichtschranken A', B', C' ausgesandten Lichtsignale werden bei Auftreffen auf die ihnen entsprechenden Reflexmarken A, B, C reflektiert und über Signalleitungen (8) an eine Meß- und Überwachungseinheit (9) weitergeleitet. Dabei sind die Lichtschranken A', B', C' von einer Montageplatte (10) am Gehäuse des Narkosegerätes (4) gehalten.

Zur weiteren Erkennung der eingestellten Konzentration des Narkosemittels ist auf der als Handrad (2) ausgebildeten Einstellvorrichtung ein weiterer Aufnehmer in Form von drei übereinanderliegenden Streifen (11) vorgesehen, auf denen jeweils eine durch weiße und schwarze Felder markierte Kodierung aufgebracht ist. Die Streifen (11) werden in ihrer Kodierung von einer Abtastvorrichtung (12) eingelesen, indem die Streifen (11) von ihnen gegenüberliegenden Lichtschranken (13) bestrahlt werden. Die Lichtstrahlen (14) werden bei Auftreffen auf einer weißen Fläche reflektiert, bei Auftreffen auf einer schwarzen Fläche absorbiert. Die daraus resultierenden Signale werden über eine Signalleitung (81) derselben Meß- und Überwachungseinheit (9) zugeführt.

In Figur 2 ist ein Teilstück der Außenfläche des Narkosegerätes (4) in dem Bereich dargestellt, in welchem die Reflexlichtschranken A', B', C' in einer Dreiecksanordnung heraustreten. Da der Narkosemittelverdunster (1) mit seiner Einstellvorrichtung (2) nicht aufgesetzt ist, sind der Aufnahmeblock (6) mit seinen Haltezapfen (19) erkennbar.

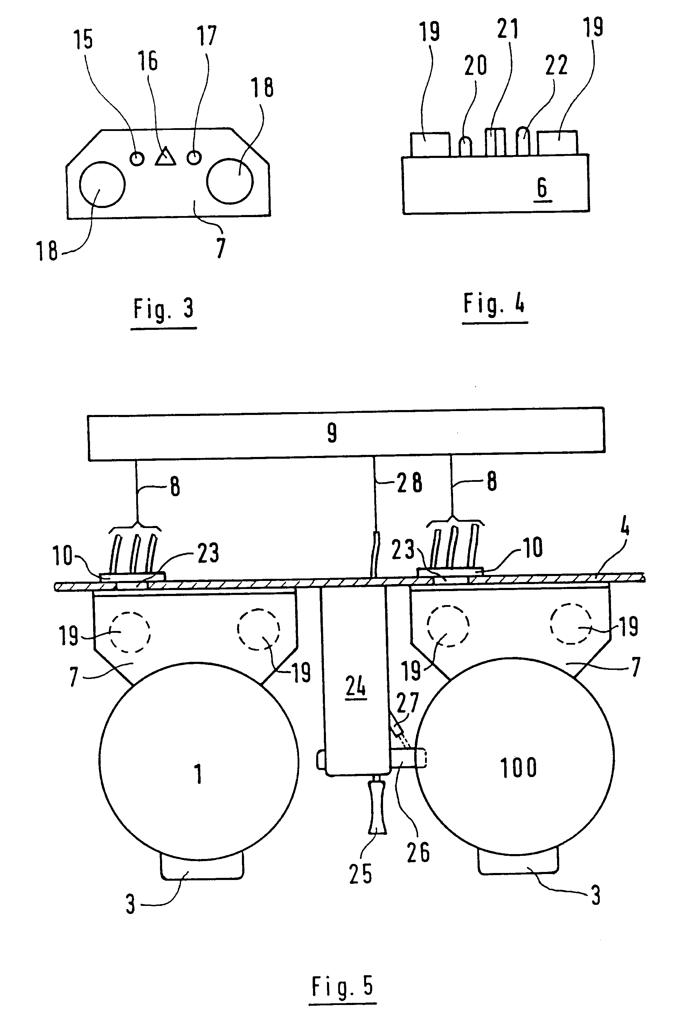

In Figur 3 ist eine weitere Ausführungsform für eine Kennung dargestellt, bei welcher in die Auflagefläche der Halterung (7) drei Ausnehmungen (15, 16, 17) unterschiedlicher Gestaltung und Tiefe eingelassen sind. Seitlich im Randbereich der Halterung (7) sind Bohrungen (18) vorgesehen, welche zur Aufnahme von zwei Haltezapfen (19) des Aufnahmeblocks (6) vorgesehen sind.

In dem in Figur (4) dargestellten Aufnahmeblock (6) sind zwischen den Haltezapfen (19) die Stifte (20, 21, 22) befestigt, welche in ihrer Formgebung und Länge den Ausnehmungen (15, 16, 17) entsprechen.

In Figur 5 sind in einer Draufsicht zwei Narkosemittelverdunster (1, 100) gezeigt, die mit ihren jeweiligen Halterungen (7) auf die entsprechenden Zapfen (19) der am Narkosegerät (4) angebrachten Aufnahmeblöcke (6) gehalten sind. Rückseitig zu den Verdunstern (1, 100) sind an der Innenfläche der Befestigungswand des Narkosegerätes (4) die jeweiligen Montageplatten (10) befestigt, in welchen die nicht dargestellten Reflexlichtschranken in einem für den jeweiligen Verdunster (1, 100) zugeordneten Muster eingelassen sind. Die Strahlung der Reflexlichtschranken A', B', C' treffen durch die Durchbrüche (23) auf die nicht dargestellten Reflexmarken A, B, C, die auf der den Lichtschranken A', B', C' zugewandten Rückseiten der Halterungen (7) angebracht sind. Zwischen beiden Verdunstern (1, 100) ist eine Sperrung (24) angeordnet, die an ihrer Stirnseite einen durch einen Schieber (25) betätigbaren Sperrhebel (26) besitzt. Durch Eingriff des Sperrhebels (26) in die Einstellvorrichtung (2) des Verdunsters (100) kann dieser nicht betätigt werden. Eine Lichtschranke (27) an der Sperrung (24) zeigt den gesperrten Zustand des Verdunsters (100) und zugleich den freigegebenen Zustand des betätigbaren Verdunsters (1) über die zugeordnete Signalleitung (28) an der Meß- und Überwachungseinheit (9) an. Wird der Sperrhebel (26) in die andere Endlage verschoben, so gerät das Ende des Sperrhebels (26) aus dem Bereich der Lichtschranke (27) und diese erhält kein Reflexlicht. Ihr daraus resultierendes Null-Signal auf der Signalleitung (28) zeigt die Sperrung des Verdunsters (1) und Freigabe des Verdunsters (100) an.

## Patentansprüche

1. Narkoseeinrichtung mit einem Narkosemittelverdunster (1), aus dem das Narkosemittel einer Einstellvorrichtung (2) zuführbar ist, von welcher aus dosierte Narkosemittelmengen an ein Narkosegerät (4) abgebbar sind, und der mit einem die Kennung für die Art des verwendeten Narkosemittels tragenden Aufnehmer (A, B, C, 15, 16, 17) versehen ist, die von einer Abtastvorrichtung (A', B', C', 20, 21, 22) identifizierbar ist, welche mittels Signalübertragungsleitungen (8) an eine Meß- und Überwachungseinheit (9) angeschlossen ist, dadurch gekennzeichnet, daß der Aufnehmer (A, B, C, 11, 15, 16, 17) an der Einstellvorrichtung (2) und/oder an dem Verdunster (1) vorgesehen ist, und daß die Abtastvorrichtung (A', B', C', 12, 20, 21, 22) an Narkosegerät (4) anzuordnen ist.

2. Narkoseeinrichtung nach Anspruch 1, dadurch gekennzeichnet, daß die Kennung mit einem Kupplungsstück (6) als Aufnehmer in Eingriff gebracht ist, in welches mehrere Ausnehmungen (15, 16, 17) unterschiedlicher Gestaltung eingelassen sind, in die mindestens ein, in seiner äußeren Form der Gestaltung der Ausnehmungen (15, 16, 17) angeglichenes Paßteil (20, 21, 22) aufnehmbar ist, wobei durch jedes in die zugehörige Ausnehmung (15, 16, 17) aufgenommene Paßteil (20, 21, 22) ein Signalgeber beeinflußbar ist.

3. Narkoseeinrichtung nach Anspruch 2, dadurch gekennzeichnet, daß die Ausnehmungen als Bohrungen (15, 16, 17) unterschiedlicher Tiefe ausgebildet sind, in welche entsprechend formgestaltete Stifte (20, 21, 22) als Paßteile eingreifen, durch die je nach Eingriffstiefe ein mit ihnen gekoppelter elektrischer Schalter als Signalgeber betätigbar ist.

4. Narkoseeinrichtung nach Anspruch 1, dadurch gekennzeichnet, daß die Kennung als eine Anordnung von Reflexlichtschranken ausgebildet ist, bei der Reflexmarken (A, B, C, 11) als Aufnehmer nach einem, für das jeweilige benutzte Narkosemittel spezifischen Muster wahlweise entweder am Narkosegerät (4) oder an der Einstellvorrichtung (2) angebracht sind, welche durch jeweils an dem anderen Geräteteil (2, 4) angebrachte Leucht- und Empfangskörper (A', B', C', 13) anstrahlbar sind, die das von den Reflexmarken (A, B, C, 11) reflektierte Licht aufnehmen.

5. Narkoseeinrichtung nach Anspruch 1, dadurch gekennzeichnet, daß die Kennung am äußeren Randbereich eines tellerförmig ausgebildeten Handrades zur Konzentrationseinstellung der Einstellvorrichtung (2) in Form eines digitalkodierten Streifens (111) ausgebildet ist, dessen Kodiermarken von einer optischen Abtasteinheit (12) durch Reflexionslicht ablesbar sind.

6. Narkoseeinrichtung nach einem der Ansprüche 1 bis 5, dadurch gekennzeichnet, daß bei Benutzung mehrerer Narkosemittelverdunster (1, 100) eine wechselseitige Sperrung (24) vorgesehen ist, durch die eine Einstellfreigabe der jeweils benutzbaren dazugehörigen Einstellvorrichtung durch eine Reflexlichtschranke (27) über eine Signalleitung (28) an der Meß- und Überwachungseinheit (9) kenntlich gemacht ist.

7. Narkoseeinrichtung nach einem der Ansprüche 1 bis 6, dadurch gekennzeichnet, daß an die Meß- und Überwachungseinheit (9) ein Kodierungsspeicher angeschlossen ist, in welchem sämtliche erlaubten Kodierungsmuster gespeichert sind und mit denen die von den Signalübertragungsstrecken (8, 81, 28) gelieferten Signale in einem Vergleicher überprüfbar sind.

8. Narkoseeinrichtung nach Anspruch 7, dadurch gekennzeichnet, daß der an die Überwachungseinheit 9 angeschlossene Kodierungsspeicher mindestens teilweise in der Einstellvorrichtung (2) des Verdunsters (1, 100) aufgenommen ist.

## Claims

1. Anaesthesia apparatus having an anaesthetic evaporator (1) from which the anaesthetic can be supplied to an adjusting arrangement (2) from which dosed quantities of anaesthetic can be delivered to an anaesthesia device (4) and which is provided with a receiver (A, B, C, 15, 16, 17) carrying the identifier of the type of anaesthetic used, which identifier can be identified by a scanning arrangement (A', B', C', 20, 21, 22) which is connected to a measuring and monitoring unit (9) by means of signal-transmitting lines (8), characterised in that the receiver (A, B, C, 11, 15, 16, 17) is provided on the adjusting arrangement (2) and/or on the evaporator (1) and in that the scanning arrangement (A', B', C', 12, 20, 21, 22) can be arranged on the anaesthesia device (4).

2. Anaesthesia apparatus according to claim 1, characterised in that the identifier is engaged with a coupling piece (6) as receiver, into which are let several recesses (15, 16, 17) of differing formation, in which recesses at least one fitting piece (20, 21, 22) can be received, the outer form of which fitting piece is adapted to the formation of the recesses (15, 16, 17), with it being possible for each fitting piece (20, 21, 22) received in the pertinent recess (15, 16, 17) to influence a signal transmitter.

3. Anaesthesia apparatus according to claim 2, characterised in that the recesses are formed as bore holes (15, 16, 17) of differing depth into which correspondingly designed pins (20, 21, 22) engage as fitting pieces by means of which, according to the depth of engagement, an electric switch coupled with them can be actuated as a signal transmitter.

4. Anaesthesia apparatus according to claim 1, characterised in that the identifier is formed as an arrangement of reflex light barriers in which case reflex marks (A, B, C, 11) are provided as receiver, according to a specific pattern for the respective anaesthetic used, selectively either on the anaesthesia device (4) or on the adjusting arrangement (2), which device or arrangement can be irradiated by means of luminous and receiving bodies (A', B', C', 13) provided respectively on the other portion of the device (2, 4) and receiving the light reflected by the reflex marks (A, B, C, 11).

5. Anaesthesia apparatus according to claim 1, characterised in that the identifier is formed on the outer edge region of a hand wheel, formed in the manner of a plate for the purpose of concentration adjustment of the adjusting arrangement (2), the identifier being in the form of a digitally coded strip (111), the code marks of which can be read off by an optical scanning unit (12) by means of reflection light.

6. Anaesthesia apparatus according to one of the claims 1 to 5, characterised in that when several anaesthetic evaporators (1, 100) are used, a two-way block (24) is provided, by means of which deblocking of the adjustment of the pertinent adjusting arrangement, which can be used respectively, is made identifiable by means of a reflex light barrier (27) by way of a signal line (28) at the measuring and monitoring unit (9).

7. Anaesthesia apparatus according to one of the claims 1 to 6, characterised in that a coding memory is connected to the measuring and monitoring unit (9), in which memory all the allowed coding patterns are stored, with which the signals supplied from the signal-transmitting sections (8, 81, 28) can be examined in a comparator.

8. Anaesthesia apparatus according to claim 7, characterised in that the coding memory connected to the monitoring unit 9 is accommodated at least in part in the adjusting arrangement (2) of the evaporator (1, 100).

## Revendications

1. Dispositif de narcose comportant un vaporisateur de narcotique (1) permettant d'envoyer le narcotique à un dispositif de réglage (2) à partir duquel des quantités dosées de narcotique peuvent être fournies à un appareil de narcose (4) et qui est pourvu d'un capteur (A, B, C, 15, 16, 17) portant l'identification du type du narcotique utilisé, qui peut être identifiée par un dispositif de détection (A', B', C', 20, 21, 22), relié à une unité de mesure et de contrôle (9), au moyen de lignes de transmission de signaux ((8), caractérisé en ce que le capteur (A, B, C, 11, 15, 16, 17) est prévu sur le dispositif de réglage (2) et/ou sur le vaporisateur (1) et en ce que le dispositif de détection (A', B', C', 12, 20, 21, 22) doit être monté sur l'appareil de narcose (4).

2. Dispositif de narcose selon la revendication 1, caractérisé en ce que l'identification est amenée en prise avec une pièce d'accouplement (6) servant de capteur, dans laquelle sont pratiqués plusieurs évidements (15, 16, 17) de forme différente dans lesquels peut être logé au moins un adaptateur (20, 21, 22) dont la forme extérieure est adaptée à celle des évidements (15, 16, 17), un transmetteur de signaux pouvant être influencé par chaque adaptateur (20, 21, 22) logé dans l'évidement correspondant (15, 16, 17).

3. Dispositif de narcose selon la revendication 2, caractérisé en ce que les évidements sont des trous (15, 16, 17) de profondeur différente dans lesquels s'engagent des broches (20, 21, 22), de forme correspondante, servant d'adaptateurs par lesquelles un interrupteur électrique, couplé avec celles-ci, servant de transmetteur de signaux, est actionné, suivant la profondeur de pénétration.

4. Dispositif de narcose selon la revendication 1, caractérisé en ce que l'identification est conçue sous la forme d'un ensemble de cellules photo-électriques réflexe, dans lequel des marques réflexe (A, B, C, 11) servant de capteurs sont pratiquées, suivant un modèle spécifique au narcotique utilisé dans chaque cas, soit sur l'appareil de narcose (4) soit sur le dispositif de réglage (2), lesquelles marques peuvent être soumises à un rayonnement par des corps lumineux et des corps récepteurs (A', B', C', 13), placés sur l'autre partie d'appareil (2, 4), qui absorbent la lumière réfléchie par des marques réflexe (A, B, C, 11).

5. Dispositif de narcose selon la revendication 1, caractérisé en ce que l'identification se présente, sur la zone de bordure extérieure d'une roue manuelle en forme de plateau, pour le réglage de la concentration du dispositif de réglage (2), sous la forme d'une bande à code numérique (111) dont les marques de codage peuvent être lues par une unité de détection optique (12), par la lumière réfléchie.

6. Dispositif de narcose selon l'une des revendications 1 à 5, caractérisé en ce que dans le cas où l'on utilise plusieurs vaporisateurs de narcotique (1, 100), il est prévu un blocage (24) réciproque par lequel une cellule à lumière réflexe (27) signale à l'unité de mesure et de contrôle (9), par une ligne de signalisation (28), un déblocage du dispositif de réglage correspondant à utiliser dans chaque cas.

7. Dispositif de narcose selon l'une des revendications 1 à 6, caractérisé en ce qu'il est raccordé à l'unité de mesure et de contrôle (9), une mémoire de codage dans laquelle sont mémorisés tous les modèles de codage autorisés et permettant de vérifier dans un comparateur les signaux fournis par les parcours de transmission de signaux (8, 81, 28).

8. Dispositif de narcose selon la revendication 7, caractérisé en ce que la mémoire de codage, reliée à l'unité de contrôle (9), est logée au moins en partie dans le dispositif de réglage (2) du vaporisateur (1, 100).
